# EUROPÄISCHE PATENTSCHRIFT

(11) **EP 4 605 917 B1**
(45) Veröffentlichungstag und Bekanntmachung des Hinweises auf die Patenterteilung: **08.07.2026**
(21) Anmeldenummer: 23794016.8
(22) Anmeldetag: 19.10.2023
(51) Int. Cl.: G08B 21/04

(54) **SICHERHEITSGERÄT UND VERFAHREN ZUM ERKENNEN EINER NOTSITUATION EINER PERSON**
SAFETY DEVICE AND METHOD FOR DETECTING AN EMERGENCY SITUATION OF A PERSON
DISPOSITIF DE SÉCURITÉ ET PROCÉDÉ DE DÉTECTION D'UNE SITUATION D'URGENCE D'UNE PERSONNE

(30) Priorität: 21.10.2022 AT 508162022
(43) Veröffentlichungstag der Anmeldung: 27.08.2025
(73) Patentinhaber: Adaptive Regelsysteme Gesellschaft m.b.H., 5020 Salzburg (AT)
(72) Erfinder: ALTENBUCHNER, Michael, 5020 Salzburg (AT); ORTNER, Andreas, 5020 Salzburg (AT)
(74) Vertreter: Weiss Patentanwalts GmbH
(86) Internationale Anmeldenummer: PCT/EP2023/079145
(87) Internationale Veröffentlichungsnummer: WO 2024/083979

(56) Entgegenhaltungen:
- CN-A- 104 408 877
- RU-C2- 2 629 795
- US-A1- 2021 295 668

## Beschreibung

Die gegenständliche Erfindung betrifft ein Sicherheitsgerät zum Erkennen einer Notsituation einer Person, die das Sicherheitsgerät trägt, wobei am Sicherheitsgerät ein Beschleunigungssensor vorgesehen ist, der eine Beschleunigung, vorzugsweise im 3-dimensionalen Raum, der das Sicherheitsgerät tragenden Person erfasst, wobei am Sicherheitsgerät eine Auswerteeinheit vorgesehen ist, die die erfasste Beschleunigung auswertet, um aus einem sich daraus ergebenden zeitlichen Verlauf der Beschleunigung eine Notsituation der Person zu erkennen und die Auswerteeinheit im Falle einer erkannten Notsituation zumindest eine konfigurierte Sicherheitsaktion auslöst. Die Erfindung betrifft auch ein zugehöriges Verfahren zum Erkennen einer Notsituation einer Person, die das Sicherheitsgerät trägt.

Wenn eine Person in einem sicherheitskritischen Umfeld arbeitet, kommen mitunter Sicherheitsgeräte zum Einsatz, die mittels Bewegungssensoren kritische Situationen bzw. Notsituationen der Person erkennen. Ein sicherheitskritisches Umfeld ist beispielsweise eine elektrische Anlage, ein elektrisches Energieübertragungssystem, eine Industrieanlage usw. Beispielsweise soll im Falle eines Kreislaufstillstandes einer Person so schnell wie möglich Hilfe gerufen werden. Es geht dabei um Minuten, ja eigentlich sogar um Sekunden, die zwischen dem Tod und der Rettung der Person liegen können. Im Falle einer anderweitig schwer verletzten Person muss auch unbedingt Hilfe gerufen werden, es ist aber nicht ganz so zeitkritisch wie bei einem Kreislaufstillstand. Das ist insbesondere wichtig, wenn die Person alleine arbeitet. Solche Notsituationen erkennt das Sicherheitsgerät, das von der Person getragen wird, oftmals mit Hilfe von Bewegungssensoren, wie beispielsweise Lage- oder Beschleunigungssensoren. Mit einem Bewegungssensor wird ein bestimmter Bewegungszustand erkannt, beispielsweise eine Regungslosigkeit, das Liegen einer Person usw. Solch ein Sicherheitsgerät löst, wenn ein bestimmter Bewegungszustand erkannt wird, einen - meist akustischen - Voralarm aus. Wenn auf den Voralarm nicht reagiert wird, beispielsweise durch eine deutliche Bewegung oder das Drücken einer Taste am Sicherheitsgerät, dann wird ein Alarm ausgelöst und Hilfe gerufen, beispielsweise durch akustische und/oder optische Signale, durch Verschicken von Textnachrichten, durch das Versenden von Emails oder durch Sprachanrufe. Die Voralarme können allerdings sehr häufig auftreten, auch dann, wenn gar keine kritische Situation vorliegt. Sitzt die Person beispielsweise am Schreibtisch oder in einer Besprechung kann ein bewegungsarmer Zustand fälschlicherweise als kritischer Bewegungszustand erkannt werden und ein Voralarm oder gar ein Alarm ausgelöst werden. Das wird früher oder später als lästig empfunden. Eine häufige Reaktion der Person, die das Sicherheitsgerät trägt ist das Ausschalten dieser Sicherheitsfunktion des Sicherheitsgeräts oder des ganzen Sicherheitsgeräts. Vergisst die Person die Sicherheitsfunktion oder das Sicherheitsgerät wieder einzuschalten, entsteht ein Gefahrenpotential, da kritische Situationen nicht mehr erkannt werden können. Eine bekannte Umgehung des Problems liegt darin, die Regungslosigkeit mit einem Lagesensor zu kombinieren. Regungslosigkeit wird nur gemeldet, wenn die Person liegt, oder sich in einem bestimmten Lagewinkel befindet. Es ist allerdings nicht garantiert, dass eine Person nach einem Sturz wirklich waagrecht zu liegen kommt, und auch nicht, dass eine bestimmte Notsituation, beispielsweise ein Herzinfarkt, in einer bestimmten Position der Person, beispielsweise bei einer mehr oder weniger sitzenden Position, eintritt.

In CN 104408877 A werden Fehlauslösungen reduziert, in dem eine Höhe mit einer Beschleunigung kombiniert wird. Ändert sich die Höhe in eine Weise, die auf einen Sturz hindeutet, so wird die Beschleunigung innerhalb einer bestimmten Zeitspanne überwacht. Nur wenn die Beschleunigung innerhalb der Zeitspanne unterhalb einer Schwelle bleibt, wird von einem Sturz ausgegangen und ein Alarm ausgelöst. Aber auch hier muss eine Veränderung der Höhe nicht zwingend mit einer Notsituation verknüpft sein bzw. können Notsituationen eintreten, die zu keiner hinreichenden Veränderung der Höhe führen.

Es besteht daher ein Bedarf, ein Sicherheitsgerät und ein Verfahren, das eine Notsituation einer das Sicherheitsgerät tragenden Person über einen Bewegungszustand erkennt, in dieser Hinsicht zu verbessern, insbesondere indem Fehlauslösungen besser unterdrückt werden.

Hierzu ist vorgesehen, dass die Auswerteeinheit eingerichtet ist, die Sicherheitsaktion für den sich ergebenden zeitlichen Verlauf der Beschleunigung nach einer Zeitdauer auszulösen und die Zeitdauer in Abhängigkeit von der erfassten Beschleunigung zu setzen.

Dadurch, dass die Zeitspanne abhängig von der Beschleunigung, also vom aktuellen Bewegungszustand, der Person gesetzt wird, kann besser darauf reagiert, dass ein gewisser Bewegungszustand vorliegt, aber trotzdem keine Notsituation für die Person gegeben ist. Bisher war eine feste Zuordnung zwischen Beschleunigung und Zeitspanne vorgesehen. Damit musste ein Kompromiss gewählt werden, was aber in gewissen Situationen eben zu Fehlauslösungen geführt hat. Durch das erfindungsgemäße Vorgehen ist die Zeitspanne von der Beschleunigung abhängig und kann damit besser an aktuelle Bewegungszustände angepasst werden, wodurch sich Fehlauslösungen besser vermeiden lassen.

Dabei ist es vorteilhaft, wenn die Auswerteeinheit die Zeitdauer umso kürzer setzt, je niedriger die aktuelle erfasste Beschleunigung ist. Dem liegt die Überlegung zugrunde, dass bei niedrigen Beschleunigungen, beispielsweise bei einer (annähernden) Regungslosigkeit, eher von einer Notsituation der Person ausgegangen werden kann, als bei größeren Beschleunigungen. Bei zu erwartenden Beschleunigungen aufgrund der normalen Bewegung einer Person, kann darauf geschlossen werden, dass gar keine Notsituation vorhanden ist. Das kann durch die Zeitdauer abgebildet werden und damit das Auslöseverhalten des Sicherheitsgerät gezielt konfiguriert werden.

In einer vorteilhaften Ausgestaltung ist vorgesehen, dass in der Auswerteeinheit ein erster Beschleunigungsschwellwert vorgesehen ist, dem eine Zeitdauer zugeordnet ist und die Auswerteeinheit die Sicherheitsaktion auslöst, wenn die erfasste Beschleunigung während der dem Beschleunigungsschwellwert zugeordneten Zeitdauer den ersten Beschleunigungsschwellwert unterschreitet. Das lässt sich besonders einfach umsetzen, weil lediglich ein Beschleunigungsschwellwert mit zugeordneter Zeitdauer vorgegeben werden müssen. Ein Vergleich der erfassten Beschleunigung mit einem Beschleunigungsschwellwert ist zudem einfach implementierbar.

Um das Auslöseverhalten zu verfeinern, kann vorgesehen sein, dass in der Auswerteeinheit ein erster Beschleunigungsschwellwert und ein zweiter Beschleunigungsschwellwert vorgesehen sind, denen jeweils eine Zeitdauer zugeordnet ist und die Auswerteeinheit die Sicherheitsaktion auslöst, wenn die erfasste Beschleunigung während der dem ersten Beschleunigungsschwellwert zugeordneten Zeitdauer den ersten Beschleunigungsschwellwert unterschreitet oder wenn die erfasste Beschleunigung während der dem zweiten Beschleunigungsschwellwert zugeordneten Zeitdauer den zweiten Beschleunigungsschwellwert unterschreitet. Dabei kann jedem Beschleunigungsschwellwert auch eine eigene Sicherheitsaktion zugewiesen sein, um auch unterschiedliche Sicherheitsaktionen auszulösen.

In einer besonders vorteilhaften Ausgestaltung ist am Sicherheitsgerät zusätzlich ein Bewegungssensor vorgesehen, der ein den Bewegungszustand charakterisierendes Bewegungssignal erfasst, das unterschiedlich von der Beschleunigung ist, und dass die Auswerteeinheit die Zeitspanne zusätzlich in Abhängigkeit vom erfassten Bewegungssignal setzt. Damit kann auf Zeitspanne noch weiter Einfluss genommen werden. Dem liegt der Gedanke zugrunde, dass eine Beschleunigung in Kombination mit einem gewissen Bewegungssignal, das beispielsweise eine Orientierung der Person widerspiegelt, unkritisch sein kann, in Kombination mit einem anderen Bewegungssignal aber sehr wohl kritisch sein kann. Das erlaubt Fehlauslösungen, noch weiter zu unterdrücken.

Das kann umgesetzt werden, indem die Auswerteeinheit zumindest einen Beschleunigungsschwellwert in Abhängigkeit vom Bewegungssignal

Die Erfindung ist in den Ansprüchen definiert.

Die gegenständliche Erfindung wird nachfolgend unter Bezugnahme auf die Figuren 1 bis 6 näher erläutert, die beispielhaft, schematisch und nicht einschränkend vorteilhafte Ausgestaltungen der Erfindung zeigen. Dabei zeigt
Fig.1 eine Person mit Sicherheitsgerät,
Fig.2 eine Person mit Sicherheitsgerät in einer Notsituation,
Fig.3 ein Beispiel eines Zusammenhanges zwischen der Beschleunigung und der Zeitdauer,
Fig.4 ein Auslösen bei Unterschreiten eines Beschleunigungsschwellwertes,
Fig.5 ein Auslösen aufgrund einer sich aus einem zeitlichen Verlauf der Beschleunigung ergebenden Zeitdauer und
Fig.6 eine Person mit Sicherheitsgerät mit einem zusätzlichen Bewegungssensor.

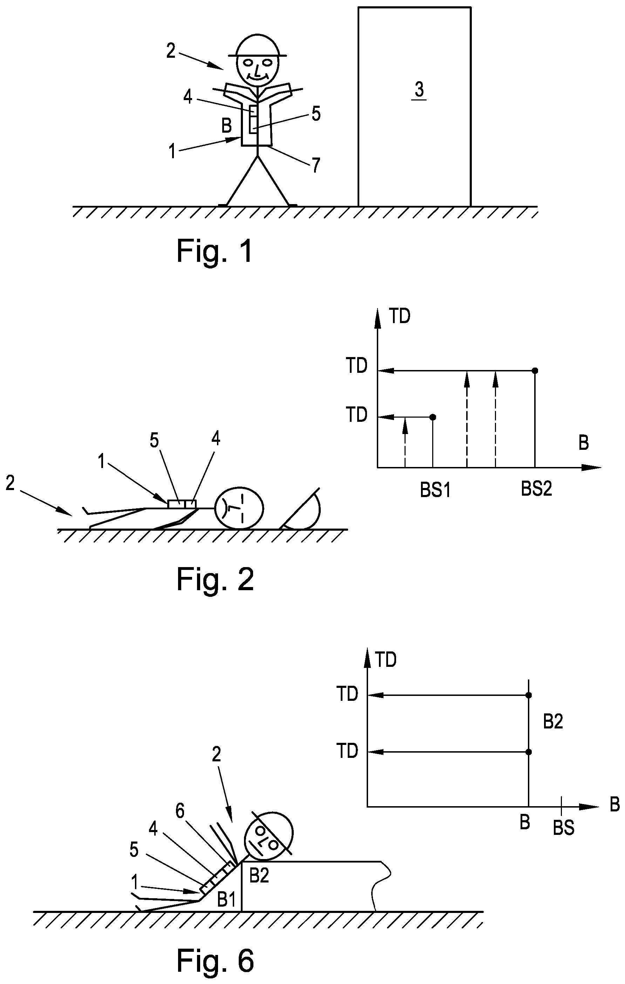

Die Erfindung betrifft ein Sicherheitsgerät 1 zum Erkennen einer Notsituation einer Person 2, die das Sicherheitsgerät 1 trägt, wie in Fig.1 dargestellt. Eine Notsituation ist eine Situation, in der Gefahr für die Gesundheit der Person 2 besteht, beispielsweise nach einem Unfall oder einen gesundheitlichen Notfall. Das Sicherheitsgerät 1 ist beispielsweise an einem Bekleidungsstück 7, beispielsweise ein Hemd, T-Shirt, Pullover, Hose usw., der Person 2 angeordnet oder in eine Tasche des Bekleidungsstücks 7 eingesteckt oder wird anderweitig von der Person 2 getragen. Das Sicherheitsgerät 1 kann auch in das Bekleidungsstück 7 integriert sein, beispielsweise als intelligentes Kleidungsstück mit integrierter Elektronik. Die Person 2 arbeitet beispielsweise an einer Anlage 3, beispielsweise ein Energieverteiler oder eine Anlage zur Erzeugung, Verteilung oder Übertragung von elektrischer Energie. Die Anlage 3 kann an einer sehr entlegenen Stelle liegen, beispielsweise ein Strommast eines Energieversorgungsnetzes.

Am Sicherheitsgerät 1 ist ein Beschleunigungssensor 4 und eine Auswerteeinheit 5 vorgesehen. Der Beschleunigungssensor 4 erfasst als Bewegungszustand der das Sicherheitsgerät 1 tragenden Person 2 eine Beschleunigung der Person 2, vorzugsweise im 3-dimensionalen Raum. Der Beschleunigungssensor 4 kann im Sicherheitsgerät 1 integriert sein, kann aber auch als separater Bauteil vorliegen und mit der Auswerteeinheit 5 in geeigneter Weise verbunden sein.

Unter Bewegungszustand wird allgemein die Position (Lage), Orientierung, Geschwindigkeit und/oder Beschleunigung der Person 2, üblicherweise im 3-dimensionalen Raum, verstanden.

Die Auswerteeinheit 5 kann eine mikroprozessorbasierte Hardware sein, auf der entsprechende Software abläuft. Die Auswerteeinheit 5 kann aber auch als integrierter Schaltkreis, wie als Field-Programmable Gate Array (FPGA) oder Anwendungsspezifische integrierte Schaltung (ASCI), ausgeführt sein. Auch andere Implementierungen sind grundsätzlich denkbar.

Der Beschleunigungssensor 4 ermittelt beispielsweise die Beschleunigungen im 3-dimensionalen Raum. Der Beschleunigungssensor 4 liefert beispielsweise drei lineare Beschleunigungswerte für die translatorische Bewegung in den drei Raumrichtungen, kann aber auch oder auch zusätzlich drei rotatorische Beschleunigungswerte für die rotatorische Bewegung liefern. Daraus kann eine lineare Beschleunigung der Person 2 im Raum ermittelt werden, aber auch alternativ oder zusätzlich ein rotativer Beschleunigungszustand. Die Beschleunigung wird üblicherweise auf eine Referenzlage oder Referenzorientierung bezogen, beispielsweise auf ein Referenzkoordinatensystem. Wenn im Folgenden von einer Beschleunigung B der Person 2 gesprochen wird, ist damit eine lineare Beschleunigung im Raum gemeint (oder auch aufgeteilt in den drei Raumrichtungen) und/oder eine rotativer Beschleunigungszustand der Person 2.

Oftmals eingesetzt zur Erfassung eines Bewegungszustands wird eine sogenannte inertiale Messeinheit. Eine inertiale Messeinheit liefert als Messwerte drei lineare Beschleunigungswerte für die translatorische Bewegung und drei Winkelgeschwindigkeiten für die Drehraten eines Körpers (hier der Person 2). Aus diesen Messwerten kann auch eine Geschwindigkeit der Person 2 im Raum (beispielsweise durch Integration der linearen Beschleunigungen) Position der Person 2 im Raum (beispielsweise durch zweimalige Integration der linearen Beschleunigungen) und eine Orientierung der Person 2 im Raum (durch Integration der Winkelgeschwindigkeiten) ermittelt werden, also ein vollständiger Bewegungszustand der Person 2. Der Beschleunigungssensor 2 ist vorteilhaft als inertiale Messeinheit ausgeführt.

Die Auswerteeinheit 5 erhält die den Bewegungszustand der Person 2 charakterisierende Beschleunigung B vom Beschleunigungssensor 4 und wertet diese aus, um über den Bewegungszustand eine Notsituation der das Sicherheitsgerät 1 tragenden Person 2 zu erkennen. Im Falle einer erkannten Notsituation löst das Sicherheitsgerät 1, z.B. die Auswerteeinheit 5, zumindest eine konfigurierte Sicherheitsaktion aus.

Die Sicherheitsaktion kann beispielsweise das Absetzen eines akustischen und/oder visuellen Alarms umfassen, aber auch die Signalisierung eines Alarms an eine im Sicherheitsgerät 1 konfigurierte Sicherheitszentrale oder Sicherheitsperson oder an einen Rettungsdienst, beispielsweise durch Verschicken einer Textnachricht, durch das Versenden einer Email oder durch einen Sprachanruf, etc. Das Sicherheitsgerät 1 umfasst damit natürlich auch eine geeignete Kommunikationsschnittstelle, um den Alarm absetzen zu können. Die Kommunikationsschnittstelle ist auch abhängig von der Art der Sicherheitsaktion und des Alarms.

Die Auswerteeinheit 5 ist eingerichtet, die Sicherheitsaktion nach einer Zeitspanne TD auszulösen, wobei die Auswerteeinheit 5 die Zeitspanne TD in Abhängigkeit von der erfassten Beschleunigung setzt. Die Zeitspanne TD wird damit an einen Bewegungszustand der Person 2 angepasst, um unerwünschte Fehlauslösungen möglichst zu vermeiden. Der erfindungsgemäße Gedanke dabei ist, dass sich eine Notsituation erst bei kleinen Beschleunigungen, also wenn sich die Person 2 wenig oder gar nicht bewegt, manifestieren wird. Treten hingegen höhere Beschleunigungen B auf, beispielsweise im Rahmen einer normalen Bewegung der Person, wird in der Regel nicht von einer Notsituation der Person 2 auszugehen sein, bzw. kann davon ausgegangen werden, dass die Person 2 noch selbst in der Lage ist Hilfe zu rufen.

Bei niedrigen Beschleunigungen B soll die Auswerteeinheit 5 die Sicherheitsaktion schneller auslösen, als bei höheren Beschleunigungen B. Daher wird die Zeitspanne TD in der Auswerteeinheit 5 bei niedrigen Beschleunigungen B kürzer gesetzt, als bei höheren Beschleunigungen B. Liegt eine bestimmte Beschleunigung B oder eine Beschleunigung kleiner einer bestimmten Beschleunigung B während der Zeitspanne TD vor, wird die Sicherheitsaktion ausgelöst. Bei höheren oder normalen Beschleunigungen B kann die Zeitdauer TD sehr groß werden, oder sogar gegen unendlich gehen.

Das lässt sich natürlich unterschiedlich realisieren, wie nachfolgend ausgeführt wird.

In einer vorteilhaften Ausgestaltung wird durch die Auswerteeinheit 5 geprüft, ob die Beschleunigung B während der Zeitspanne TD einen vorgegebenen Beschleunigungsschwellwert BS1 unterschreitet.

Die Zeitspanne TD kann dem Beschleunigungsschwellwert BS1 zugeordnet sein, beispielsweise durch Konfiguration der Auswerteeinheit 5. Unterschreitet die erfasste Beschleunigung B den Beschleunigungsschwellwert BS1, wird geprüft, ob die Beschleunigung B während der Zeitspanne TD, die dem Beschleunigungsschwellwert BS1 zugeordnet ist, unterschritten wird. Dabei können natürlich auch mehrere unterschiedliche Beschleunigungsschwellwerte BS1, BS2 vorgesehen sein, denen jeweils eine Zeitdauer TD zugeordnet ist.

Beispielsweise kann die Person 2 durch einen Unfall regungslos am Boden liegen, wie in Fig.2 dargestellt. In einem solchen Bewegungszustand werden vom Beschleunigungssensor 4 nur sehr geringe Beschleunigungen B erfasst, beispielsweise kleiner eines ersten Beschleunigungsschwellwerts BS1 von 25 m-g (Milli-g, also Tausendstel der Erdbeschleunigung g). Selbst im Falle einer Regungslosigkeit der Person 2 ist davon auszugehen, dass kleine Beschleunigungen B erfasst werden, entweder wegen dem Messrauschen des Beschleunigungssensors 4 oder wegen Vibrationen des Bodens auf dem die Person 2 liegt. Daher sollte ein unterster Beschleunigungsschwellwert BS1 solche Einflüsse berücksichtigen. Das deutet aufgrund der Regungslosigkeit auf einen schwerwiegenden Vorfall hin und auf eine gravierende Notsituation der Person 2. In diesem Fall kann die Zeitdauer TD des Unterschreitens kurz gesetzt werden, beispielsweise auf 60 s (Sekunden). Ändert sich an diesem Bewegungszustand während der Zeitdauer TD nichts, wird von der Auswerteeinheit 5 die konfigurierte Sicherheitsaktion ausgelöst, beispielsweise das Absetzen eines Alarms, beispielsweise an einen Rettungsdienst. Wird hingegen eine Beschleunigung B kleiner eines zweiten Beschleunigungsschwellwerts BS2 von 100 m-g erfasst, kann von einer geringen Bewegung der Person 2, beispielsweise aufgrund der Atmung, geschlossen werden. In diesem Fall kann die zweite Zeitdauer TD2 des Unterschreitens etwas länger gesetzt werden, beispielsweise auf 300 s (Sekunden). Über dem zweiten Beschleunigungsschwellwerts BS2 wird beispielsweise gar keine Sicherheitsaktion oder nur die Alarmierung eines Kollegen ausgelöst, oder es können noch weitere Beschleunigungsschwellwerte über dem zweiten Beschleunigungsschwellwert BS2 vorgegeben sein.

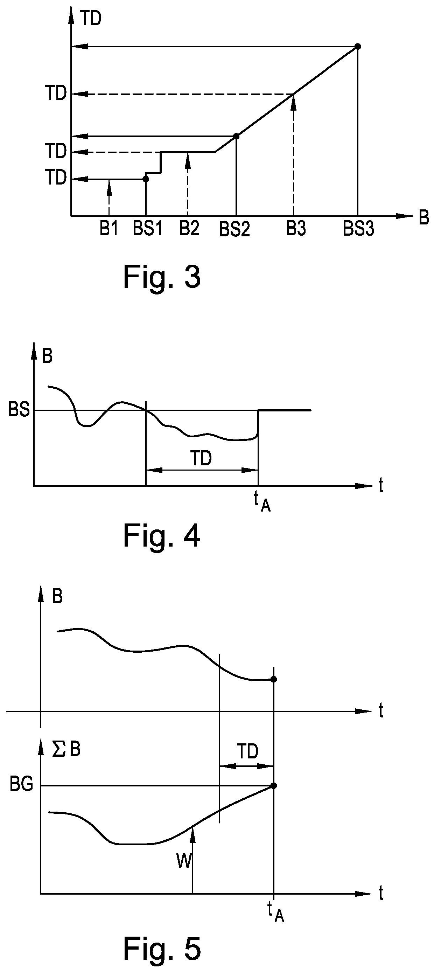

Fig.3 zeigt ein anderes Ausführungsbeispiel eines Zusammenhangs zwischen der Beschleunigung und der Zeitdauer TD. Dargestellt sind drei verschiedene erste Bewegungssignale B1, B2, B3, denen jeweils eine Zeitdauer TD zugeordnet ist. In diesem Beispiel gibt es einen kontinuierlichen (hier linearen) Bereich und diskrete Bereiche der Zuordnung. Für kleineste Beschleunigungen B, kleiner einem Beschleunigungsschwellwert BS1 ist wieder dem Beschleunigungsschwellwert BS1 eine Zeitdauer TD zugeordnet, die für Beschleunigungen B1 unterhalb vom Beschleunigungsschwellwert BS1 zu berücksichtigen ist. Für größere Beschleunigungen B3 ist die Zeitdauer TD kontinuierlich von der Beschleunigung B3 abhängig, d.h., dass jeder Beschleunigung B3 gemäße eines definierten Zusammenhanges eine Zeitdauer TD zugeordnet ist. Ist die Beschleunigung B3 unterhalb des Beschleunigungsschwellwerts BS3, dann wird die Zeitdauer TD auf den Wert gesetzt, der sich aus der Beschleunigung B3 ergibt. Für mittlere Beschleunigungen B2 ist eine Mischung aus einer diskreten und kontinuierlichen Zuordnung vorgesehen, aus der sich die Zeitdauer TD ableitet.

Der Zusammenhang zwischen der Beschleunigung B und der Zeitdauer TD, beispielsweise wie in Fig.2 oder Fig.3 dargestellt, ist in geeigneter Weise in der Auswerteeinheit 5 hinterlegt, beispielsweise in Tabellenform, als mathematische Funktion, diskret oder kontinuierlich.

In dieser Ausgestaltung wird anhand einer aktuellen erfassten Beschleunigung B eine Zeitdauer TD gesetzt und dann überprüft, ob die Beschleunigung B in dieser Zeitspanne einen bestimmten zeitlichen Verlauf aufweist, beispielsweise kleiner einem bestimmten Beschleunigungsschwellwert BS ist. Anders ausgedrückt, hat im Auslösezeitpunkt t_{A} der Sicherheitsaktion der vergangene Beschleunigungsverlauf in der vergangenen Zeitspanne TD somit einen bestimmten zeitlichen Verlauf angenommen, beispielsweise kleiner einem bestimmten Beschleunigungsschwellwert BS. Die vergangene Zeitdauer TD ist die unmittelbar vor dem Auslösezeitpunkt t_{A} liegende Zeitspanne. Das ist beispielhaft in Fig.4 dargestellt, die die Beschleunigung B über der Zeit t darstellt. Im Auslösezeitpunkt t_{A} des Sicherheitsgeräts 1 war die Beschleunigung B während der Zeitdauer TD, die wie oben beschrieben von der Beschleunigung B abhängig ist, kleiner einem Beschleunigungsschwellwert BS.

In einer anderen Ausgestaltung wird die Zeitdauer TD nicht als solche ermittelt, sondern die Zeitdauer TD ergibt sich aus dem zeitlichen Beschleunigungsverlauf und wird auf diese Weise in der Auswerteeinheit 5 gesetzt. Die Zeitdauer TD ist damit aber ebenfalls abhängig von der erfassten Beschleunigung B.

In einer möglichen Ausführung, nach Fig.5, wird das Sicherheitsgerät 1 aufgrund einer sich aus einem zeitlichen Verlauf der Beschleunigung B ergebenden Zeitdauer TD ausgelöst.

Beispielsweise wird aus der zu jedem Zeitpunkt (vorzugsweise in zeitdiskreten Abtastschritten) erfassten Beschleunigung B eine Beschleunigungswirkung W ermittelt, beispielsweise als Kehrwert 1/B der Beschleunigung B, gegebenenfalls auch gewichtet, beispielsweise um kleine Beschleunigungen noch stärker zu berücksichtigen. Die Beschleunigungswirkungen W in jedem Zeitpunkt werden über ein vorgegebenes Zeitfenster zu einer Beschleunigungssumme ΣB aufaddiert. Das Zeitfenster ist eine vorgegebene Beobachtungszeitspanne, kann aber auch die gesamte Zeitdauer seit Einschalten des Sicherheitsgeräts 1 sein. Gleichzeitig wird in jedem Zeitpunkt von der aktuellen Beschleunigungssumme ΣB eine vorgegebene Beschleunigungswirkung W subtrahiert, wobei die Beschleunigungssumme ΣB nicht negativ werden kann. Für große Beschleunigungen B ist der Kehrwert klein und die Beschleunigungssumme ΣB wird nieder oder sogar Null sein, insbesondere, weil ja auch in jedem Zeitschritt eine Beschleunigungswirkung W subtrahiert wird. Ist hingegen eine aktuelle Beschleunigung B klein, so ist der Kehrwert groß und die Beschleunigungssumme ΣB steigt stark an, insbesondere, wenn die Beschleunigung B für einen längeren Zeitraum klein bleibt, beispielsweise im Falle einer Regungslosigkeit der Person 2. Übersteigt die Beschleunigungssumme ΣB einen vorgegebenen Grenzwert BG, wird die Sicherheitsaktion ausgelöst. Daraus ergibt sich die Zeitdauer TD als die Zeitspanne vor dem Auslösezeitpunkt t_{A}, in der die Beschleunigung B niedrige Werte hatte. Die Wirkung ist damit die gleiche, wie in der Ausgestaltung nach Fig. 4.

Es ist offensichtlich, dass die Beschleunigung B in einer solchen Ausgestaltung auch anders als mit einer Beschleunigungssumme ΣB bewertet werden kann. Beispielsweise könnte ein gleitender Mittelwert über ein Zeitfenster ermittelt werden. Ist der Mittelwert kleiner einem Grenzwert könnte die Sicherheitsaktion ausgelöst werden. Ebenso könnte die Fläche unter der Beschleunigung während eines Zeitfensters, beispielsweise als Integral, bewertet werden.

In dieser Ausgestaltung ergibt sich die Zeitdauer TD somit aus einem vergangenen Beschleunigungsverlauf, wobei die Zeitdauer TD umso kürzer wird, je niedriger die Beschleunigung B im vergangenen Beschleunigungsverlauf ist. Der vergangene Beschleunigungsverlauf ist dabei der zeitliche Verlauf der Beschleunigung B in einem vorgegebenen Zeitfenster vor dem aktuellen Zeitpunkt.

Die Zeitdauer TD kann zusätzlich von einem zweiten Bewegungssignal B2, das unterschiedlich zum ersten Bewegungssignal B1 ist, abhängig sein, wie anhand von Fig.6 erläutert wird.

In diesem Ausführungsbeispiel umfasst das Sicherheitsgerät 1 einen Bewegungssensor 6, der einen Bewegungszustand der das Sicherheitsgerät 1 tragenden Person 2 erfasst. Der Bewegungssensor 6 erfasst ein Bewegungssignal B2, das unterschiedlich von der erfassten Beschleunigung B ist. Das zweite Bewegungssignal B2 wird der Auswerteeinheit 5 übermittelt und darin ausgewertet.

Der Bewegungssensor 6 ist beispielsweise ein Lagesensor, der ein Bewegungssignal B2 bereitstellt, das die Orientierung der Person 2 im 3-dimensionalen Raum charakterisiert.

Wenn der Beschleunigungssensor 4 als inertiale Messeinheit ausgeführt ist, die auch eine Position oder Orientierung der Person 2 im Raum liefern kann, kann der Beschleunigungssensor 4 natürlich gleichzeitig auch als Bewegungssensor 6 fungieren. Der Beschleunigungssensor 4 und der Bewegungssensor 6 sind damit in einem Sensor integriert ausgeführt.

Wenn die erfasste Beschleunigung B einen vorgegebenen Beschleunigungsschwellwert BS unterschreitet, kann zusätzlich das weitere Bewegungssignal B2 geprüft werden. Ist das Bewegungssignal B2 innerhalb eines vorgegebenen Signalbereichs, wird eine bestimmte Zeitdauer TD gesetzt. Ist das Bewegungssignal B2 außerhalb des vorgegebenen Signalbereichs, wird eine andere Zeitdauer TD gesetzt, wie in Fig.6 angedeutet. Dabei sind natürlich auch mehrere Signalbereiche möglich, denen jeweils eine bestimmte Zeitdauer TD zugeordnet ist.

Im Falle eines Lagesensors als zweiten Bewegungssensor 6 kann als Signalbereich (Lagebereich) beispielsweise ein Bereich von -45° bis +45° (bezogen auf eine Horizontale) gesetzt sein. Wird nun durch die Beschleunigung B ein Beschleunigungsschwellwert BS unterschritten (im obigen Ausführungsbeispiel z.B. 100 m-g), wird die Zeitdauer TD auf 60 s gesetzt, wenn zusätzlich eine Orientierung der Person 2 innerhalb des Lagebereichs festgestellt wird. Ist die Orientierung außerhalb, wird die Zeitdauer TD auf 300 s gesetzt.

Die Zeitdauer TD ist damit nicht nur von der erfassten Beschleunigung B abhängig, sondern auch vom weiteren Bewegungssignal B2.

Ferner ist es denkbar, dass der zumindest eine Beschleunigungsschwellwert BS vom zweiten Bewegungssignal B2 abhängig ist. In diesem Fall ist es möglich, andere Beschleunigungsschwellwerte BS für die Beschleunigung B vorzusehen, in Abhängigkeit von einem aktuellen Bewegungssignal B2.

Beispielsweise könnte ein anderer Beschleunigungsschwellwerte BS vorgesehen sein, wenn die Person 2 liegt, gegenüber einem Bewegungszustand, in dem die Person 2 steht oder sitzt. Das Liegen der Person 2 kann beispielsweise wieder durch einen Lagebereich von -45° bis +45° festgestellt werden.

## Patentansprüche

1. Sicherheitsgerät zum Erkennen einer Notsituation einer Person (2), die das Sicherheitsgerät (1) trägt, wobei am Sicherheitsgerät (1) ein Beschleunigungssensor (4) vorgesehen ist, der eine Beschleunigung (B), vorzugsweise im 3-dimensionalen Raum, der das Sicherheitsgerät (1) tragenden Person (2) erfasst, wobei am Sicherheitsgerät (1) eine Auswerteeinheit (5) vorgesehen ist, die die erfasste Beschleunigung (B) auswertet, um aus einem sich daraus ergebenden zeitlichen Verlauf der Beschleunigung (B) eine Notsituation der Person (2) zu erkennen und die Auswerteeinheit (5) im Falle einer erkannten Notsituation zumindest eine konfigurierte Sicherheitsaktion auslöst, wobei die Auswerteeinheit (5) eingerichtet ist, die Sicherheitsaktion für den sich ergebenden zeitlichen Verlauf der Beschleunigung (B) nach einer Zeitdauer (TD) auszulösen, **dadurch gekennzeichnet, dass** die Auswerteeinheit (5) eingerichtet ist, die Zeitdauer (TD) in Abhängigkeit von der erfassten Beschleunigung (B) zu setzen.

2. Sicherheitsgerät nach Anspruch 1, **dadurch gekennzeichnet, dass** die Auswerteeinheit (5) die Zeitdauer (TD) umso kürzer setzt, je niedriger die aktuelle erfasste Beschleunigung (B) ist.

3. Sicherheitsgerät nach Anspruch 1 oder 2, **dadurch gekennzeichnet, dass** in der Auswerteeinheit (5) ein erster Beschleunigungsschwellwert (BS1) vorgesehen ist, dem eine Zeitdauer (TD) zugeordnet ist und die Auswerteeinheit (5) die Sicherheitsaktion auslöst, wenn die erfasste Beschleunigung (TD) während der dem Beschleunigungsschwellwert (BS1) zugeordneten Zeitdauer (TD) den ersten Beschleunigungsschwellwert (BS1) unterschreitet.

4. Sicherheitsgerät nach Anspruch 1 oder 2, **dadurch gekennzeichnet, dass** in der Auswerteeinheit (5) ein erster Beschleunigungsschwellwert (BS1) und ein zweiter Beschleunigungsschwellwert (BS2) vorgesehen sind, denen jeweils eine Zeitdauer (TD) zugeordnet ist und die Auswerteeinheit (5) die Sicherheitsaktion auslöst, wenn die erfasste Beschleunigung (B) während der dem ersten Beschleunigungsschwellwert (BS1) zugeordneten Zeitdauer (TD) den ersten Beschleunigungsschwellwert (BS1) unterschreitet oder wenn die erfasste Beschleunigung (B) während der dem zweiten Beschleunigungsschwellwert (BS2) zugeordneten Zeitdauer (TD) den zweiten Beschleunigungsschwellwert (BS2) unterschreitet.

5. Sicherheitsgerät nach Anspruch 1 oder 2, **dadurch gekennzeichnet, dass** in der Auswerteeinheit (5) ein erster Beschleunigungsschwellwert (BS1) und ein zweiter Beschleunigungsschwellwert (BS2) vorgesehen sind, denen jeweils eine Zeitdauer (TD) zugeordnet ist und die Auswerteeinheit (5) eine erste Sicherheitsaktion auslöst, wenn die erfasste Beschleunigung (B) während der dem ersten Beschleunigungsschwellwert (BS1) zugeordneten Zeitdauer (TD) den ersten Beschleunigungsschwellwert (BS1) unterschreitet oder die Auswerteeinheit (5) eine zweite Sicherheitsaktion auslöst, wenn die erfasste Beschleunigung (B) während der dem zweiten Beschleunigungsschwellwert (BS2) zugeordneten Zeitdauer (TD) den zweiten Beschleunigungsschwellwert (BS2) unterschreitet.

6. Sicherheitsgerät nach Anspruch 1 oder 2, **dadurch gekennzeichnet, dass** die Auswerteeinheit (5) eingerichtet ist, eine Sicherheitsaktion aufgrund einer sich aus einem zeitlichen Verlauf der Beschleunigung (B) ergebenden Zeitdauer (TD) auszulösen.

7. Sicherheitsgerät nach einem der Ansprüche 1 bis 6, **dadurch gekennzeichnet, dass** am Sicherheitsgerät (1) ein Bewegungssensor (6) vorgesehen ist, der ein den Bewegungszustand charakterisierendes Bewegungssignal (B2) erfasst, das unterschiedlich von der Beschleunigung (B) ist, und dass die Auswerteeinheit (5) die Zeitdauer (TD) zusätzlich in Abhängigkeit vom erfassten Bewegungssignal (B2) setzt.

8. Sicherheitsgerät nach Anspruch 7, **dadurch gekennzeichnet, dass** die Auswerteeinheit (5) zumindest einen Beschleunigungsschwellwert (BS1, BS2) in Abhängigkeit vom Bewegungssignal (B2) setzt.

9. Sicherheitsgerät nach Anspruch 7 oder 8, **dadurch gekennzeichnet, dass** der Bewegungssensor (6) ein Lagesensor ist und das Bewegungssignal (B2) eine Orientierung der Person (2), vorzugsweise im 3-dimensionalen Raum, angibt.

10. Verfahren zum Erkennen einer Notsituation einer Person (2) mit einem Sicherheitsgerät, das die Person (2) trägt, wobei mit einem Beschleunigungssensor (4) am Sicherheitsgerät (1) eine Beschleunigung (B), vorzugsweise im 3-dimensionalen Raum, der das Sicherheitsgerät (1) tragenden Person (2) erfasst wird und die erfasste Beschleunigung (B) mit einer Auswerteeinheit (5) ausgewertet wird, um aus einem sich aus der erfassten Beschleunigung (B) ergebenden zeitlichen Verlauf der Beschleunigung (B) eine Notsituation der Person (2) zu erkennen und im Falle einer erkannten Notsituation zumindest eine konfigurierte Sicherheitsaktion von der Auswerteeinheit (5) ausgelöst wird, wobei die Auswerteeinheit (5) die Sicherheitsaktion für den sich ergebenden zeitlichen Verlauf der Beschleunigung (B) nach einer Zeitdauer (TD) auslöst, **dadurch gekennzeichnet, dass** die Auswerteeinheit (5) die Zeitdauer (TD) in Abhängigkeit von der erfassten Beschleunigung (B) setzt.

11. Verfahren nach Anspruch 10, **dadurch gekennzeichnet, dass** die Zeitdauer (TD) umso kürzer gesetzt wird, je niedriger die aktuelle erfasste Beschleunigung (B) ist.

12. Verfahren nach Anspruch 10 oder 11, **dadurch gekennzeichnet, dass** die Sicherheitsaktion ausgelöst wird, wenn die erfasste Beschleunigung (B) während einer einem vorgegebenen Beschleunigungsschwellwert (BS1) zugeordneten Zeitdauer (TD) den ersten Beschleunigungsschwellwert (BS1) unterschreitet.

13. Verfahren nach Anspruch 10 oder 11, **dadurch gekennzeichnet, dass** die Sicherheitsaktion ausgelöst wird, wenn die erfasste Beschleunigung (B) während einer einem vorgegebenen ersten Beschleunigungsschwellwert (BS1) zugeordneten ersten Zeitdauer (TD) den ersten Beschleunigungsschwellwert (BS1) unterschreitet oder wenn die erfasste Beschleunigung (B) während einer einem vorgegebenen zweiten Beschleunigungsschwellwert (BS2) zugeordneten zweiten Zeitdauer (TD) den zweiten Beschleunigungsschwellwert (BS2) unterschreitet.

14. Verfahren nach Anspruch 10 oder 11, **dadurch gekennzeichnet, dass** eine erste Sicherheitsaktion ausgelöst wird, wenn die erfasste Beschleunigung (B) während einer einem vorgegebenen ersten Beschleunigungsschwellwert (BS1) zugeordneten ersten Zeitdauer (TD) den ersten Beschleunigungsschwellwert (BS1) unterschreitet oder eine zweite Sicherheitsaktion ausgelöst wird, wenn die erfasste Beschleunigung (B) während einer einem vorgegebenen zweiten Beschleunigungsschwellwert (BS2) zugeordneten zweiten Zeitdauer (TD) den zweiten Beschleunigungsschwellwert (BS2) unterschreitet.

15. Verfahren nach Anspruch 10 oder 11, **dadurch gekennzeichnet, dass** eine Sicherheitsaktion aufgrund einer sich aus einem zeitlichen Verlauf der Beschleunigung (B) ergebenden Zeitdauer (TD) ausgelöst wird.

16. Verfahren nach einem der Ansprüche 10 bis 15, **dadurch gekennzeichnet, dass** mit einem Bewegungssensor (6) ein den Bewegungszustand der Person (2) charakterisierendes Bewegungssignal (B2) erfasst wird, das unterschiedlich von der Beschleunigung (B) ist, und dass die Zeitdauer (TD) zusätzlich in Abhängigkeit vom erfassten Bewegungssignal (B2) gesetzt wird.

17. Verfahren nach Anspruch 16, **dadurch gekennzeichnet, dass** zumindest ein Beschleunigungsschwellwert (BS1) in Abhängigkeit vom Bewegungssignal (B2) gesetzt wird.

## Claims

1. Safety device for detecting an emergency situation of a person (2) wearing the safety device (1), wherein an acceleration sensor (4) is provided on the safety device (1) which detects an acceleration (B), preferably in 3-dimensional space, of the person (2) wearing the safety device (1), wherein an analysis unit (5) is provided on the safety device (1) which analyzes the detected acceleration (B) to detect an emergency situation of the person (2) from an acceleration (B) time curve produced therefrom, and wherein the analysis unit (5) initiates at least one configured safety action in the event of a detected emergency situation, wherein the analysis unit (5) is configured to initiate the safety action for the produced acceleration (B) time curve after a period of time (TD), **characterized in that** the analysis unit (5) is configured to set said period of time (TD) on the basis of the detected acceleration (B).

2. Safety device according to claim 1, **characterized in that** the analysis unit (5) sets the period of time (TD) to be shorter the lower the currently detected acceleration (B) is.

3. Safety device according to claim 1 or 2, **characterized in that** a first acceleration threshold value (BS1) is provided in the analysis unit (5), to which a period of time (TD) is assigned, and the analysis unit (5) initiates the safety action, if the detected acceleration (TD) falls below the first acceleration threshold value (BS1) during the period of time (TD) assigned to the acceleration threshold value (BS1).

4. Safety device according to claim 1 or 2, **characterized in that** a first acceleration threshold value (BS1) and a second acceleration threshold value (BS2) are provided in the analysis unit (5), to each of which a period of time (TD) is assigned, and wherein the analysis unit (5) initiates the safety action, if the detected acceleration (B) falls below the first acceleration threshold value (BS1) during the period of time (TD) assigned to the first acceleration threshold value (BS1) or if the detected acceleration (B) falls below the second acceleration threshold value (BS2) during the period of time (TD) assigned to the second acceleration threshold value (BS2).

5. Safety device according to claim 1 or 2, **characterized in that** a first acceleration threshold value (BS1) and a second acceleration threshold value (BS2) are provided in the analysis unit (5), to each of which a period of time (TD) is assigned, and where the analysis unit (5) initiates a first safety action, if the detected acceleration (B) falls below the first acceleration threshold value (BS1) during the period of time (TD) assigned to the first acceleration threshold value (BS1), or the analysis unit (5) initiates a second safety action, if the detected acceleration (B) falls below the second acceleration threshold value (BS2) during the period of time (TD) assigned to the second acceleration threshold value (BS2).

6. Safety device according to claim 1 or 2, **characterized in that** the analysis unit (5) is configured to initiate a safety action on the basis of a period of time (TD) resulting from an acceleration (B) time curve.

7. Safety device according to any of claims 1 to 6, **characterized in that** a movement sensor (6) is provided on the safety device (1), which detects a movement signal (B2) characterizing the movement state, which signal is different from the acceleration (B), and **in that** the analysis unit (5) sets the period of time (TD) additionally on the basis of the detected movement signal (B2).

8. Safety device according to claim 7, **characterized in that** the analysis unit (5) sets at least one acceleration threshold value (BS1, BS2) on the basis of the movement signal (B2).

9. Safety device according to claim 7 or 8, **characterized in that** the movement sensor (6) is a position sensor and the movement signal (B2) indicates an orientation of the person (2), preferably in 3-dimensional space.

10. Method for detecting an emergency situation of a person (2) with a safety device worn by the person (2), wherein an acceleration (B), preferably in 3-dimensional space, of the person (2) wearing the safety device (1) is detected by means of an acceleration sensor (4) on the safety device (1), and the detected acceleration (B) is analyzed by an analysis unit (5) to detect an emergency situation of the person (2) from the acceleration (B) time curve produced from the detected acceleration (B), and wherein at least one configured safety action is initiated by the analysis unit (5) in the event of a detected emergency situation, wherein the analysis unit (5) initiates the safety action for the produced acceleration (B) time curve after a period of time (TD), **characterized in that** the analysis unit (5) sets the period of time (TD) on the basis of the detected acceleration (B).

11. Method according to claim 10, **characterized in that** the period of time (TD) is set to be shorter the lower the currently detected acceleration (B) is.

12. Method according to claim 10 or 11, **characterized in that** the safety action is initiated, if the detected acceleration (B) falls below the first acceleration threshold value (BS1) during a period of time (TD) assigned to a predetermined acceleration threshold value (BS1).

13. Method according to claim 10 or 11, **characterized in that** the safety action is initiated, if the detected acceleration (B) falls below the first acceleration threshold value (BS1) during a first period of time (TD) assigned to a predetermined first acceleration threshold value (BS1) or if the detected acceleration (B) falls below the second acceleration threshold value (BS2) during a second period of time (TD) assigned to a predetermined second acceleration threshold value (BS2).

14. Method according to claim 10 or 11, **characterized in that** a first safety action is initiated, if the detected acceleration (B) falls below the first acceleration threshold value (BS1) during a first period of time (TD) assigned to a predetermined first acceleration threshold value (BS1), or a second safety action is initiated, if the detected acceleration (B) falls below the second acceleration threshold value (BS2) during a second period of time (TD) assigned to a predetermined second acceleration threshold value (BS2).

15. Method according to claim 10 or 11, **characterized in that** a safety action is initiated on the basis of a period of time (TD) resulting from an acceleration (B) time curve.

16. Method according to any of claims 10 to 15, **characterized in that** a movement sensor (6) detects a movement signal (B2) characterizing the movement state of the person (2), which signal is different from the acceleration (B), and **in that** the period of time (TD) is set additionally on the basis of the detected movement signal (B2).

17. Method according to claim 16, **characterized in that** at least one acceleration threshold value (BS1) is set on the basis of the movement signal (B2).

## Revendications

1. Appareil de sécurité permettant de reconnaître une situation d'urgence d'une personne (2) qui porte l'appareil de sécurité (1), dans lequel un capteur d'accélération (4) est prévu sur l'appareil de sécurité (1), lequel détecte une accélération (B), de préférence dans un espace tridimensionnel, de la personne (2) portant l'appareil de sécurité (1), dans lequel une unité d'évaluation (5) est prévue sur l'appareil de sécurité (1), laquelle évalue l'accélération (B) détectée pour reconnaître une situation d'urgence de la personne (2) à partir d'une évolution temporelle résultante de l'accélération (B) et l'unité d'évaluation (5) déclenche au moins une action de sécurité configurée dans le cas d'une situation d'urgence reconnue, dans lequel l'unité d'évaluation (5) est configurée pour déclencher l'action de sécurité pour l'évolution temporelle résultante de l'accélération (B) après une durée (TD), **caractérisé en ce que** l'unité d'évaluation (5) est configurée pour fixer la durée (TD) en fonction de l'accélération (B) détectée.

2. Appareil de sécurité selon la revendication 1, **caractérisé en ce que** l'unité d'évaluation (5) fixe la durée (TD) à une valeur d'autant plus courte que l'accélération (B) actuelle détectée est faible.

3. Appareil de sécurité selon la revendication 1 ou 2,
**caractérisé en ce qu'**est prévue dans l'unité d'évaluation (5) une première valeur seuil d'accélération (BS1) à laquelle est associée une durée (TD) et l'unité d'évaluation (5) déclenche l'action de sécurité si l'accélération (TD) détectée est inférieure à la première valeur seuil d'accélération (BS1) pendant la durée (TD) associée à la valeur seuil d'accélération (BS1).

4. Appareil de sécurité selon la revendication 1 ou 2, **caractérisé en ce que** sont prévues dans l'unité d'évaluation (5) une première valeur seuil d'accélération (BS1) et une seconde valeur seuil d'accélération (BS2) auxquelles est associée respectivement une durée (TD) et l'unité d'évaluation (5) déclenche l'action de sécurité si l'accélération (B) détectée est inférieure à la première valeur seuil d'accélération (BS1) pendant la durée (TD) associée à la première valeur seuil d'accélération (BS1) ou si l'accélération (B) détectée est inférieure à la seconde valeur seuil d'accélération (BS2) pendant la durée (TD) associée à la seconde valeur seuil d'accélération (BS2).

5. Appareil de sécurité selon la revendication 1 ou 2, **caractérisé en ce que** sont prévues dans l'unité d'évaluation (5) une première valeur seuil d'accélération (BS1) et une seconde valeur seuil d'accélération (BS2) auxquelles est associée respectivement une durée (TD) et l'unité d'évaluation (5) déclenche une première action de sécurité si l'accélération (B) détectée est inférieure à la première valeur seuil d'accélération (BS1) pendant la durée (TD) associée à la première valeur seuil d'accélération (BS1) ou l'unité d'évaluation (5) déclenche une seconde action de sécurité si l'accélération (B) détectée est inférieure à la seconde valeur seuil d'accélération (BS2) pendant la durée (TD) associée à la seconde valeur seuil d'accélération (BS2).

6. Appareil de sécurité selon la revendication 1 ou 2, **caractérisé en ce que** l'unité d'évaluation (5) est configurée pour déclencher une action de sécurité sur la base d'une durée (TD) résultant d'une évolution temporelle de l'accélération (B).

7. Appareil de sécurité selon l'une des revendications 1 à 6,
**caractérisé en ce qu'**est prévu sur l'appareil de sécurité (1) un capteur de mouvement (6) qui détecte un signal de mouvement (B2) caractérisant l'état de mouvement, lequel signal de mouvement est différent de l'accélération (B),
et **en ce que** l'unité d'évaluation (5) fixe la durée (TD) en outre en fonction du signal de mouvement (B2) détecté.

8. Appareil de sécurité selon la revendication 7, **caractérisé en ce que** l'unité d'évaluation (5) fixe au moins une valeur seuil d'accélération (BS1, BS2) en fonction du signal de mouvement (B2).

9. Appareil de sécurité selon la revendication 7 ou 8,
**caractérisé en ce que** le capteur de mouvement (6) est un capteur de position et le signal de mouvement (B2) indique une orientation de la personne (2), de préférence dans un espace tridimensionnel.

10. Procédé permettant de reconnaître une situation d'urgence d'une personne (2) avec un appareil de sécurité que porte la personne (2), dans lequel une accélération (B), de préférence dans l'espace tridimensionnel, de la personne (2) portant l'appareil de sécurité (1) est détectée avec un capteur d'accélération (4) sur l'appareil de sécurité (1) et l'accélération (B) détectée est évaluée avec une unité d'évaluation (5) pour reconnaître une situation d'urgence de la personne (2) à partir d'une évolution temporelle de l'accélération (B) résultant de l'accélération (B) détectée et, dans le cas d'une situation d'urgence reconnue, au moins une action de sécurité configurée est déclenchée par l'unité d'évaluation (5), dans lequel l'unité d'évaluation (5) déclenche l'action de sécurité pour l'évolution temporelle résultante de l'accélération (B) après une durée (TD), **caractérisé en ce que** l'unité d'évaluation (5) fixe la durée (TD) en fonction de l'accélération (B) détectée.

11. Procédé selon la revendication 10, **caractérisé en ce que** la durée (TD) est fixée à une valeur d'autant plus courte que l'accélération (B) actuelle détectée est faible.

12. Procédé selon la revendication 10 ou 11, **caractérisé en ce que** l'action de sécurité est déclenchée si l'accélération (B) détectée est inférieure à une première valeur seuil d'accélération (BS1) pendant une durée (TD) associée à la valeur seuil d'accélération (BS1) prédéfinie.

13. Procédé selon la revendication 10 ou 11, **caractérisé en ce que** l'action de sécurité est déclenchée si l'accélération (B) détectée est inférieure à une première valeur seuil d'accélération (BS1) pendant une première durée (TD) associée à la première valeur seuil d'accélération (BS1) prédéfinie ou si l'accélération (B) détectée est inférieure à une seconde valeur seuil d'accélération (BS2) pendant une seconde durée (TD) associée à la seconde valeur seuil d'accélération (BS2) prédéfinie.

14. Procédé selon la revendication 10 ou 11, **caractérisé en ce qu'**une première action de sécurité est déclenchée si l'accélération (B) détectée est inférieure à une première valeur seuil d'accélération (BS1) pendant une première durée (TD) associée à la première valeur seuil d'accélération (BS1) prédéfinie ou une seconde action de sécurité est déclenchée si l'accélération (B) détectée est inférieure à une seconde valeur seuil d'accélération (BS2) pendant une seconde durée (TD) associée à la seconde valeur seuil d'accélération (BS2) prédéfinie.

15. Procédé selon la revendication 10 ou 11, **caractérisé en ce qu'**une action de sécurité est déclenchée sur la base d'une durée (TD) résultant d'une évolution temporelle de l'accélération (B).

16. Procédé selon l'une des revendications 10 à 15,
**caractérisé en ce qu'**un signal de mouvement (B2) caractérisant l'état de mouvement de la personne (2) est détecté par un capteur de mouvement (6), lequel signal de mouvement est différent de l'accélération (B)**, et en ce que** la durée (TD) est fixée en outre en fonction du signal de mouvement (B2) détecté.

17. Procédé selon la revendication 16, **caractérisé en ce qu'**au moins une valeur seuil d'accélération (BS1) est fixée en fonction du signal de mouvement (B2).
